(19) Europäisches Patentamt
European Patent Office
Office européen des brevets

(11) **EP 4 205 692 B1**

## (12) EUROPEAN PATENT SPECIFICATION

(45) Date of publication and mention
of the grant of the patent:
**20.11.2024 Bulletin 2024/47**

(21) Application number: **22215274.6**

(22) Date of filing: **21.12.2022**

(51) International Patent Classification (IPC):
*A61B 90/30* (2016.01)     *A61B 90/00* (2016.01)
*H05B 45/22* (2020.01)     *A61B 1/06* (2006.01)
*A61B 1/00* (2006.01)     *A61B 1/05* (2006.01)

(52) Cooperative Patent Classification (CPC):
**A61B 90/30; A61B 1/05; A61B 1/0638;**
**A61B 1/0655; A61B 90/361; H05B 45/22;**
A61B 1/00165; A61B 2090/306; H05B 47/125

(54) **SYSTEMS AND METHODS FOR GENERATING METAMERICALLY MATCHED ILLUMINATION**

SYSTEME UND VERFAHREN ZUR ERZEUGUNG EINER METAMERISCH ANGEPASSTEN BELEUCHTUNG

SYSTÈMES ET PROCÉDÉS DE GÉNÉRATION D'ÉCLAIRAGE MIS EN CORRESPONDANCE DE MANIÈRE MÉTAMÉRIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **30.12.2021 US 202163294929 P**

(43) Date of publication of application:
**05.07.2023 Bulletin 2023/27**

(73) Proprietor: **Karl Storz Imaging, Inc.**
**Goleta, CA 93117 (US)**

(72) Inventor: **Bormet, Jonathan**
**Goleta, CA (US)**

(74) Representative: **Weidner Stern Jeschke**
**Patentanwälte Partnerschaft mbB**
**Wilhelm-Herbst-Straße 5**
**28359 Bremen (DE)**

(56) References cited:
**US-A1- 2005 143 662     US-A1- 2008 065 345**
**US-A1- 2015 044 098     US-A1- 2019 191 511**
**US-A1- 2020 305 259     US-A1- 2021 075 978**

• **KIMIYOSHI MIYATA: "Development of Practical Investigation System for Cultural Properties Based on a Projector-Camera System", PROC. SPIE 7241, COLOR IMAGING XIV: 724104, vol. 7241, 19 January 2009 (2009-01-19), XP040493902, DOI: 10.1117/12.805770**

**Description**

CROSS-REFERENCE TO RELATED APPLICATIONS

The present application claims the benefit of and priority, under 35 U.S.C. § 119(e), to U.S. Provisional Application Serial No. 63/294,929, filed on December 30, 2021, entitled "SYSTEMS AND METHODS FOR GENERATING METAMERICALLY MATCHED ILLUMINATION."

BACKGROUND

The present disclosure is generally directed to illumination, in particular, toward illumination that results in user perception of metamerically matched illumination and reflection of illumination.

Areas in which work is being performed can benefit from illumination. In particular, surgeries or surgical procedures often benefit from illumination on or around a surgical site. The illumination may sequence between spectrally distinct illuminants which may appear to the user as a broad spectrum white light and an excitation illuminant having a narrower spectrum perceived by the user as a hue of light. The alternation between illuminants may result in a visible flicker (strobing effect) of the illuminant and/or the surgical site that can be visually jarring and distract or annoy a surgeon and/or others in the operating suite. This undesired strobing effect may result in surgeon dissatisfaction or may negatively impact the surgery or surgical procedure.

US 2020/0305259 A1 discloses an endoscope system with an endoscope, wherein a light control unit causes light source units to emit light by applying a pulse current value with a pulse width larger than a predetermined pulse width in order to emit white illumination light towards an illumination fiber of the endoscope. Afterwards, the light source control unit shifts to a strobe observation mode by causing the respective light sources to emit light by applying a pulse current with the predetermined pulse width thereto while synchronizing an observation mode of a light source device with a vibration frequency of sound input from an outside, in order to synchronize the color balanced light with the vibration frequency of vocal cords captured by a sound input device.

US 2008/0065345 A1 describes a method and a system for controlling an illumination system, such as a backlight system for a display, comprising a plurality of LEDs of the colors red, green and blue as light sources and a single optical sensor adapted to sense the light output from each of the light source channels and, optionally, a temperature sensor providing the sensing temperature of the LEDs. Sensor values are used by a controller to compensate changes in the intensity outputs of the LEDs varying according to factors such as temperature and age.

US 2021/0075978 A1 describes a method for visible and/or near-infrared imaging of a sample, wherein three different LEDs (blue, green and red) with visible light emission and one LED with near-infrared emission are used and emitting wavelengths of the LEDs are configured to match the light absorbance of oxygen carrying hemoglobin and deoxygenated hemoglobin for measuring the hemoglobin oxygen saturation. Likewise, US 2005/0143662 A1 concerns an imaging device for pulse oximetry and therefore for measuring the oxygen concentration of blood, wherein illuminating light consists of alternating pulses of radiant energy by two LEDs with a red and an infrared wavelength controlled by a controller.

SUMMARY

The problem is solved by a system to illuminate a first surgical site, the system comprising: a light source; a processor and a controller that determines a metamerically matched illumination, the metamerically matched illumination including a first set of illuminants and a second set of illuminants that, when temporally sequenced and at least partially superimposed, appears as a continuous white light; and an imaging device in communication with the controller that by means of the light source emits the metamerically matched illumination on the first surgical site.

Issues associated with the above-mentioned strobing effect may be addressed by embodiments of the present disclosure. According to at least one embodiment, each of the illumination spectra creating the illumination of a surgical site through a surgical instrument may be manipulated such that the apparent color (e.g., the color appearing to a user of the surgical instrument) of each illuminant appears identical (or largely similar) when integrated by the human visual system (e.g., rods and cones in the human eye). In other words, the amplitude and/or wavelength of light being generated and projected from the surgical instrument may be adjusted such that, when viewed by the eyes of the user, the light appears as, for example, a white light (or other color of light), despite an underlying change in illumination spectra. In some embodiments, the illumination spectra may be manipulated such that the strobing effect is diminished, minimized, or otherwise eliminated as viewed by the user.

Unless otherwise specified, "metamerically matching", "metamerism", "metameric", and/or variations thereof means the phenomenon of producing spectra that are spectrally distinct but visually indistinct. Stated differently, the spectra contain different wavelengths of light that, when integrated, appear to a user as visually indistinct from one another (e.g., a temporally white light).

In some embodiments, the production of metamerically matching spectra may occur using a system that analyzes the spectrum, models the human visual system, and calibrates the light source based on the analysis and modeling. Additionally or alternatively, the system may monitor tristimulus values (e.g., values of the red, green, and blue light present in the illumination) of the illuminant real-time using a colorimeter integrated within the light source, or by using the endoscopic camera. Such determinations of the tristimulus values may enable the system to update or maintain the correct illuminants to ensure a metamerically matched output. The real-time monitoring may beneficially enable the system to compensate for fluctuations within the light source, such as those associated with unit-by-unit variability or varying operating conditions such as, for example, Light Emitting Diode (LED) drive strength, temperature, and/or duty cycle.

In some embodiments, the active monitoring approach may include a control loop that manipulates the spectral components of each illuminant in sequence to compensate for unknown spectral reflectance of the surgical scene. For example, in an exoscopic use case, it may be preferable to minimize the flicker or strobing effects from light reflected off anatomical elements being viewed (e.g., patient anatomy), rather than flicker or strobing effects associated with the direct viewing of the illuminant (e.g., the illuminant generated by the light source and exiting the exoscope). In this use case, the color information collected by the camera (e.g., a camera in the exoscope) could be used to stabilize the object colors (based on, for example, the product of the illuminant and the scene reflectance).

In some embodiments, exoscopic scenes, as well as other surgical environments, may contain multiple distinct tissue types and surgical implements within the scene, making it difficult to stabilize all object colors simultaneously. That is, certain portions of the scene may no longer be a metameric match. In such scenes, locally targeted illumination may be combined with the metameric matching to allow certain portions of the scene to be metamerically matched. By associating spatially distinct regions within the scene with distinct light-emitting elements (e.g., using individual light fibers, using individual LEDs in an array, using individual pixels in a projector, etc.), a plurality of lighting elements each with independent spectral control of the illuminant sequence could be configured to stabilize several object colors in several spatially distinct regions within the scene.

In one exemplary aspect according to at least one embodiment of the present disclosure to illuminate a first surgical site, a system comprises: a controller that determines a metamerically matched illumination, the metamerically matched illumination including a first set of illuminants and a second set of illuminants that, when temporally sequenced and at least partially superimposed, appears to a user as a continuous white light; and an imaging device in communication with the controller that emits the metamerically matched illumination on the first surgical site.

Any of the embodiments herein, wherein the first set of illuminants comprises a first red light spectra, a first green light spectra, and a first blue light spectra.

Any of the embodiments herein, further comprising: a display device configured to display the illuminated first surgical site.

Any of the embodiments herein, further comprising: a processor; and a memory storing instructions thereon that, when executed by the processor, cause the processor to: determine a first amplitude of the first red light spectra, a first amplitude of the first green light spectra, and a first amplitude of the first blue light spectra to generate a first white light.

Any of the embodiments herein, wherein the system further comprises: a light source, the light source designed to: receive the first amplitudes from the processor; and generate, based on the first amplitudes, the first white light.

Any of the embodiments herein, further comprising: a light guide, the light guide configured to guide the continuous white light from the light source to the imaging device.

Any of the embodiments herein, wherein the first set of illuminants and the second set of illuminants appear as the continuous white light to a user.

Any of the embodiments herein, wherein the first surgical site includes a first anatomical element.

Any of the embodiments herein, wherein the second set of illuminants comprises a second red light spectra, a second green light spectra, and a second blue light spectra.

Any of the embodiments herein, wherein the instructions further cause the processor to:

determine a second amplitude of the second red light spectra, a second amplitude of the second green light spectra, and a second amplitude of the second blue light spectra to generate a second white light.

Any of the embodiments herein, wherein the light source is further designed to: receive the second amplitudes from the processor; and generate, based on the second amplitudes, the second white light.

Any of the embodiments herein, wherein the instructions further cause the processor to: temporally sequencing from the first set of illuminants to the second set of illuminants.

Use of the system including any one or more of the embodiments disclosed herein to illuminate the first surgical site.

In another aspect according to at least one embodiment of the present disclosure to illuminate a first surgical site, a system comprises: a controller that determines a metamerically matched reflectance, the metamerically matched reflectance including a first set of illuminants and a second set of illuminants that, when temporally sequenced, at least partially superimposed, and shone on the first surgical site, reflectively appear to a user as a continuous white light; and an imaging device in communication with the controller that emits the first set of illuminants and the second set of illuminants on the first surgical site.

Any of the embodiments herein, wherein the first set of illuminants comprises a first red light spectra, a first green light spectra, and a first blue light spectra.

Any of the embodiments herein, further comprising: a display device configured to display the illuminated first surgical site.

Any of the embodiments herein, further comprising: a processor; and a memory storing instructions thereon that, when executed by the processor, cause the processor to: determine a first amplitude of the first red light spectra, a first amplitude of the first green light spectra, and a first amplitude of the first blue light spectra to generate a first white light.

Any of the embodiments herein, wherein the system further comprises: a light source, the light source designed to: receive the first amplitudes from the processor; and generate, based on the first amplitudes, the first white light.

Any of the embodiments herein, further comprising: a light guide, the light guide configured to guide the continuous white light from the light source to the imaging device.

Any of the embodiments herein, wherein the first surgical site includes a first anatomical element.

Any of the embodiments herein, wherein the second set of illuminants comprises a second red light spectra, a second green light spectra, and a second blue light spectra.

Any of the embodiments herein, wherein the instructions further cause the processor to: determine a second amplitude of the second red light spectra, a second amplitude of the second green light spectra, and a second amplitude of the second blue light spectra to generate a second white light.

Any of the embodiments herein, wherein the light source is further designed to: receive the second amplitudes from the processor; and generate, based on the second amplitudes, the second white light.

Any of the embodiments herein, wherein the instructions further cause the processor to: temporally sequencing from the first set of illuminants to the second set of illuminants.

Use of the system including any one or more of the embodiments disclosed herein to illuminate the first surgical site.

In a further aspect according to at least one embodiment of the present disclosure, a method for illuminating a first surgical site comprises: determining an illumination requirement, wherein an illumination requirement is a metamerically matched illumination or reflectance; determining, based on the illumination requirement, a first set of illuminants; determining, based on the illumination requirement, a second set of illuminants; generating the first set of illuminants or the second set of illuminants using a light source; causing the first set of illuminants to be shone on a first illumination site; temporally sequencing from the first set of illuminants to the second set of illuminants; and causing the second set of illuminants to be shone on the first illumination site, wherein the first set of illuminants and the second set of illuminants appear as a continuous white light.

Any of the embodiments herein, further comprising: rendering, to a first display, the first illumination site illuminated by the first set of illuminants or the second set of illuminants.

Any of the embodiments herein, wherein the determining the illumination requirement comprises: receiving, from a first imaging device, video data.

Any of the embodiments herein, further comprising: generating, using a light source, the first set of illuminants or the second set of illuminants.

Any of the embodiments herein, wherein the first set of illuminants and the second set of illuminants appear to a user as a continuous white light.

In yet another aspect according to at least one embodiment of the present disclosure, a system comprises: a means for determining a metamerically matched illumination, the metamerically matched illumination including a first set of illuminants and a second set of illuminants that, when temporally sequenced, appears to a user as a continuous white light; and a means for generating the metamerically matched illumination on a first surgical site.

Any of the embodiments herein, further comprising: a means for displaying the illuminated first surgical site.

Any of the embodiments herein, further comprising: a means for determining a first amplitude of a first red light spectra, a first amplitude of a first green light spectra, and a first amplitude of a first blue light spectra to generate the continuous white light; and a means for generating, based on the first amplitudes, the continuous white light.

Any of the embodiments herein, further comprising: a means for guiding the continuous white light from the means for generating the continuous white light to the means for generating the metamerically matched illumination on the first surgical site.

Still another embodiment shown and described herein, wherein a controller determines a metamerically matched illumination, the metamerically matched illumination including a first set of illuminants and a second set of illuminants that, when temporally sequenced, appears to a user as a continuous white light, and wherein an imaging device generates the metamerically matched illumination on a first surgical site.

Any of the embodiments herein, wherein the first set of illuminants comprises a first red light spectra, a first green light spectra, and a first blue light spectra.

Any of the embodiments herein, wherein a display device is configured to display the illuminated first surgical site.

Any of the embodiments herein, further comprising: a processor; and a memory storing instructions thereon that, when executed by the processor, cause the processor to: determine a first amplitude of the first red light spectra, a first amplitude of the first green light spectra, and a first amplitude of the first blue light spectra to generate the continuous white light.

Any of the embodiments herein, wherein the system further comprises: a light source, the light source designed to: receive the first amplitudes from the processor; and generate, based on the first amplitudes, the continuous white light.

Any aspect and/or embodiment in combination with any one or more other embodiments.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the embodiments or features as disclosed herein.

It is to be appreciated that any feature described herein can be claimed in combination with any other feature(s) as described herein, regardless of whether the features come from the same described embodiment.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The controller can comprise the processor or the system comprises the processor as a separate component or as part of any other component of the system. Furthermore, the system can also comprise two or more processors.

In yet another embodiment, the systems and methods of this disclosure can be implemented in conjunction with a special purpose computer, a programmed microprocessor or microcontroller and peripheral integrated circuit element(s), an ASIC or other integrated circuit, a digital signal processor, a hard-wired electronic or logic circuit such as discrete element circuit, a programmable logic device or gate array such as PLD, PLA, FPGA, PAL, special purpose computer, any comparable means, or the like. In general, any device(s) or means capable of implementing the methodology illustrated herein can be used to implement the various aspects of this disclosure. Exemplary hardware that can be used for the present disclosure includes computers, handheld devices, telephones (e.g., cellular, Internet enabled, digital, analog, hybrids, and others), and other hardware known in the art. Some of these devices include processors (e.g., a single or multiple microprocessors), memory, nonvolatile storage, input devices, and output devices. Furthermore, alternative software implementations including, but not limited to, distributed processing or component/object distributed processing, parallel processing, or virtual machine processing can also be constructed to implement the methods described herein.

In yet another embodiment, the disclosed methods may be readily implemented in conjunction with software using object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with this disclosure is dependent on the speed and/or efficiency requirements of the system, the particular function, and the particular software or hardware systems or microprocessor or microcomputer systems being utilized.

In yet another embodiment, the disclosed methods may be partially implemented in software that can be stored on a storage medium, executed on programmed general-purpose computer with the cooperation of a controller and memory, a special purpose computer, a microprocessor, or the like. In these instances, the systems and methods of this disclosure can be implemented as a program embedded on a personal computer such as an applet, JAVA® or CGI script, as a resource residing on a server or computer workstation, as a routine embedded in a dedicated measurement system, system component, or the like. The system can also be implemented by physically incorporating the system and/or method into a software and/or hardware system.

The term "automatic" and variations thereof, as used herein, refers to any process or operation, which is typically continuous or semi-continuous, done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material."

Aspects of the present disclosure may take the form of an embodiment that is entirely hardware, an embodiment that is entirely software (including firmware, resident software, micro-code, etc.) or an embodiment combining software and hardware aspects that may all generally be referred to herein as a "circuit," "module," or "system." Any combination of one or more computer-readable medium(s) may be utilized. The computer-readable medium may be a computer-readable signal medium or a computer-readable storage medium.

A computer-readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer-readable storage medium may be any tangible medium that can contain or store a program for use by or in connection with an instruction execution system, apparatus, or device.

A computer-readable signal medium may include a propagated data signal with computer-readable program code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device. Program code embodied on a computer-readable medium may be transmitted using any appropriate medium, including, but not limited to, wireless, wireline, optical fiber cable, RF, etc., or any suitable combination of the foregoing.

The terms "determine," "calculate," "compute," and variations thereof, as used herein, are used interchangeably and include any type of methodology, process, mathematical operation or technique.

The details of one or more embodiments of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

BRIEF DESCRIPTION OF THE DRAWINGS

These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below:

Fig. 1      is a block diagram of a system according to at least one embodiment of the present disclosure;
Fig. 2A     shows images under broad spectrum illumination;
Fig. 2B     shows the images under metamerically matched illumination;
Fig. 3      shows a chromaticity diagram according to at least one embodiment of the present disclosure;
Fig. 4A     shows a first illumination sequence according to at least one embodiment of the present disclosure;
Fig. 4B     shows a second illumination sequence according to at least one embodiment of the present disclosure;
Fig. 5A     shows various narrow spectrum wavelength peaks within spectral regions associated with visible colors corresponding to according to at least one embodiment of the present disclosure;
Fig. 5B     shows an illuminated image according to at least one embodiment of the present disclosure;
Fig. 6A     shows a surgical object illuminated using different illuminants according to at least one embodiment of the present disclosure;
Fig. 6B     shows multiple surgical objects illuminated using different illuminants according to at least one embodiment of the present disclosure;
Fig. 7      shows illuminant spectra according to at least one embodiment of the present disclosure; and

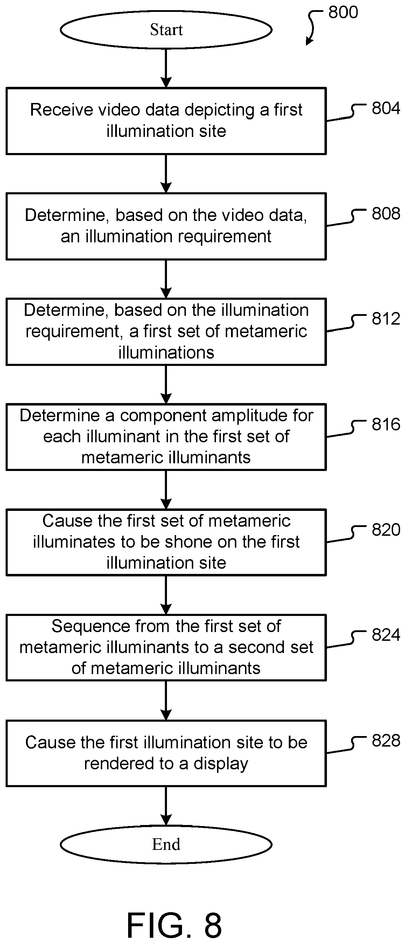

Fig. 8      is a flowchart according to at least one embodiment of the present disclosure.

DETAILED DESCRIPTION

It should be understood that various embodiments disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain embodiments of this disclosure are described as being performed by a single module or unit for purposes of clarity, it should be understood that the techniques of this disclosure may be performed by a combination of units or modules associated with, for example, a computing device and/or a medical device.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Embodiments of the present disclosure provide technical solutions to one or more of the problems of user-perceived (1) strobing or flickering light effects in an environment, such as a surgical environment and (2) strobing or flickering light effects associated with reflected light off an object, such as a surgical object.

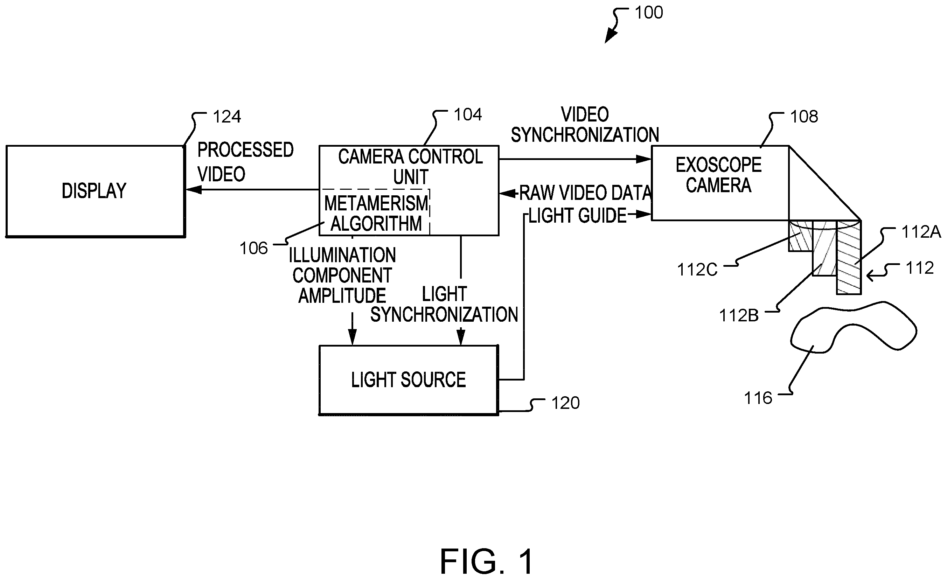

Turning first to Fig. 1, a block diagram of a system 100 according to at least one embodiment of the present disclosure is shown. The system 100 may be used to illuminate and image a surgical site (and/or one or more anatomical elements contained therein); determine two or more metamerically matched illuminations; process video data and/or render processed video data to a display; sequence through one or more illuminants; and/or carry out one or more other aspects of one or more of the methods discussed herein. The system 100 comprises a camera control unit 104, an exoscope camera 108, a light source 120, and a display 124. Systems according to other embodiments of the present disclosure may comprise more or fewer components than the system 100. For instance, the system 100 may comprise additional cameras or imaging devices, medical instruments for use during a surgery or surgical procedure, combinations thereof, and/or the like.

The camera control unit 104 comprises a processor (not shown), one or more metamerism algorithms 106, a communication interface (not shown), a memory (not shown), and a user interface (not shown). The camera control unit 104 is configured to receive information (e.g., raw video data or image data), process the information, and output the processed information in a format that is usable by one or more other components of the system 100 (e.g., processed video data rendered to the display 124).

The processor of the camera control unit 104 may be any processor described herein or any similar processor such as a graphics processing unit (GPU). The processor may be configured to execute instructions stored in a memory or received from a source external to the camera control unit 104, which instructions may cause the processor to carry out one or more computing steps utilizing or based on data received from any one or more components of the system 100 (e.g., the exoscope camera 108, the light source 120, the display 124, etc.) and/or from sources external to the system 100. The processor may be able to access other components of the system 100, such as the memory. In some embodiments, each component of the system 100 may comprise a separate processor, with each processor of each component in communication with one another for the purposes of, for example, communication to carry out one or more steps of the method 800, the sharing of computational power, or for any other purpose.

The memory accessed by the processor may be or comprise RAM, DRAM, SDRAM, other solid-state memory, any memory described herein, or any other tangible, non-transitory memory for storing computer-readable data and/or instructions. In at least one embodiment, the memory is positioned within the camera control unit 104. The memory may store information or data useful for completing, for example, any step of the method 800 described herein, or of any other methods. The memory may store, for example, the metamerism algorithm 106. Additionally or alternatively, the memory may store other algorithms or instructions that, when executed by the processor, enable the determination of metamerically matched spectra. The algorithms or other instructions may be organized into one or more applications, modules, packages, layers, or engines.

The communication interface of the camera control unit 104 may be used for receiving image/video data or other information from an external source (such as the exoscope camera 108, the light source 120, the display 124, and/or any other system or component not part of the system 100), and/or for transmitting instructions, images, or other information to an external system or device (e.g., to another camera control unit 104, the exoscope camera 108, the light source 120, the display 124, and/or any other system or component not part of the system 100). The communication interface may comprise one or more wired interfaces (e.g., a USB port, an Ethernet port, etc.) and/or one or more wireless transceivers or interfaces (e.g., Bluetooth®). In some embodiments, the communication interface may be useful for enabling the camera control unit 104 to communicate with one or more other camera control units 104. In some embodiments, the camera control unit 104 may remotely (e.g., wirelessly) access the communication interface directly or indirectly (e.g., accessing a proxy), and then use the communication interface to communicate with one or more components of the system 100. In some embodiments, each component of the system 100 (e.g., the exoscope camera 108, the light source 120, the display 124, etc.) may contain a communication interface, such that each component of the system 100 can communicate with one another.

The user interface may be or comprise a touchscreen, a monitor, a television, a screen, a keyboard, a mouse, a trackball, and/or any other device for receiving information from a user (e.g., a physician, a surgeon, a member of a surgical staff) and/or for providing information to a user. The user interface may be used, for example, to receive a user selection or other user input regarding any step of any method described herein (e.g., a method 800). Notwithstanding the foregoing, any required input for any step of any method described herein may be generated automatically by the system 100 (e.g., by the camera control unit 104 or another component of the system 100) or received by the system 100 from a source external to the system 100. In some embodiments, the user interface may be useful to allow a surgeon or other user to modify instructions to be executed by the camera control unit 104 (e.g., instructions for executing the metamerism algorithm 106) according to one or more embodiments of the present disclosure, and/or to modify or adjust a setting of other information displayed on the user interface.

The light source 120 generates light that can be used during a surgery or surgical procedure. The light source 120 may include a light bulb, an LED, a laser, combinations thereof, etc. or any other light-generating or light-emitting element capable of generating light at different wavelengths and/or amplitudes. In some embodiments, the light source 120 may comprise multiple light-emitting elements (e.g., an array of LEDs). In such embodiments, each individual light-emitting element may be independent of the other light-emitting elements and be capable of generating a different wavelength of light. In one embodiment, the light source 120 is capable of producing at least three different colors of light (e.g., red light or light between about 650 and 750 nanometers (nm) in wavelength, green light or light between about 500 and 565 nm in wavelength, blue light or light between about 450 nm and 485 nm in wavelength, combinations thereof, and/or the like). In some embodiments, the light source 120 may be controlled or operated by the camera control unit 104. In other embodiments, the light source 120 may include a processor that receives data or instructions from the camera control unit 104 and/or the metamerism algorithm 106 and uses such data or instructions to generate light. For example, the light source 120 may receive illumination component amplitudes and/or wavelengths from the metamerism algorithm 106 and generate light based on such input. Similarly, the light source 120 may receive light synchronization instructions from the camera control unit 104 (e.g., timing information for each LED in an array of LEDs such that each frame of the exoscope camera 108 displays the correct color combination) and use such information to produce the desired light at the desired moment in time. The light source 120 may pass the light into the exoscope camera 108 using, for example, a light guide. The light guide may be or comprise a transparent material (e.g., glass, plastic, etc.) and filaments that pass the light from the light source 120 to the exoscope camera 108. In some embodiments, the light guide may be or comprise fiber optic cable or some other dielectric waveguide that directs the light emitted from the light source 120 through the exoscope camera 108 to illuminate the anatomical element 116.

The exoscope camera 108 may be an imaging device connected to an exoscope or other surgical instrument capable of receiving light from a light source 120 and emitting the light, such that, for example, the anatomical element 116 is illuminated with the light generated by the light source 120. In some embodiments, the exoscope camera 108 may be operable to emit a continuous sequence of light generated by the light source 120. For example, the exoscope camera 108 may be configured to operate with an open shutter, or with a rolling shutter that continuously alternates between open and shut so as to capture successive images. In some embodiments, the light source 120 may generate light such that each frame captured by the exoscope camera 108 is illuminated by a different color or different combinations of spectrally distinct illuminants (e.g., frame one includes red illuminant, frame two includes green illuminant, frame three includes blue illuminant, etc.).

Additionally or alternatively, the system 100 may comprise an endoscope camera. The endoscope camera is attached to the end of an endoscope or other surgical instrument and is configured to be inserted into the patient during the course of a surgery or surgical procedure. For instance, the endoscope camera may be inserted into an incision in the patient, such that the endoscope camera can capture images of and/or illuminate the underlying tissue. Similar to the exoscope camera 108, the endoscope camera may be capable of receiving light from the light source 120 and emit the light to, for example, illuminate a surgical site during a surgery or surgical procedure. While an exoscope camera 108 and an endoscope camera are discussed herein, additional or alternative cameras or imaging devices may be implemented, and the use of the exoscope camera 108 and/or the endoscope camera is in no way limited.

The exoscope camera 108 (or, in some embodiments, an endoscope camera and/or other camera) may emit one or more illuminants 112 onto the anatomical element 116 or, more generally, onto a surgical object or surgical site. The illuminants 112 may comprise a first illuminant 112A, a second illuminant 112B, and a third illuminant 112C. Each of the first illuminant 112A, the second illuminant 112B, and the third illuminant 112C may be a different user-perceived color (e.g., a different wavelength of light) and/or have a different amplitudes associated therewith. In one embodiment, the first illuminant 112A may comprise a red light, the second illuminant 112B may comprise green light, and the third illuminant 112C may comprise blue light. The illuminants 112 may be combined in any manner to generate an infinite number of colors of light. In some embodiments, the first illuminant 112A, the second illuminant 112B, and/or the third illuminant 112C may be temporally cycled (e.g., the first illuminant 112A is turned on while the second illuminant 112B and the third illuminant 112C are turned off, then the second illuminant 112B is turned on and the first illuminant 112A is turned off) such that a desired color of light is shone on the anatomical element 116. The illumination shone on the anatomical element 116 may be captured by the exoscope camera 108 or other component of the system 100 (e.g., a different imaging device), with the raw video data depicting the illuminated anatomical element 116 passed to the camera control unit 104. The camera control unit 104 may, for example, use the raw video data to update the metamerism algorithm 106, to process and send the video data to the display 124 to be displayed to a user (e.g., a surgeon), to be stored in a database, or for any other purpose.

In some embodiments, the first illuminant 112A, the second illuminant 112B, and the third illuminant 112C may be metamerically matched illuminants. For instance, the metamerism algorithm 106 may receive raw and/or processed video data from the camera control unit 104, and, in combination with an estimation of the human visual system (e.g., based on an estimation performed by the metamerism algorithm 106), determine a first set of illuminants 112A, 112B, and 112C required such that when the first set of illuminants exit the exoscope camera 108 and shine on the anatomical element 116, the combined illumination or reflected light from the anatomical element in the surgical site appears to the user as a substantially continuous color or a smoothly transitioning color in view of a prior set or subsequent set of illuminants, presented in a continuous sequence while imaging the surgical site, rather than a perceived flickering or strobing of illumination or reflected light.

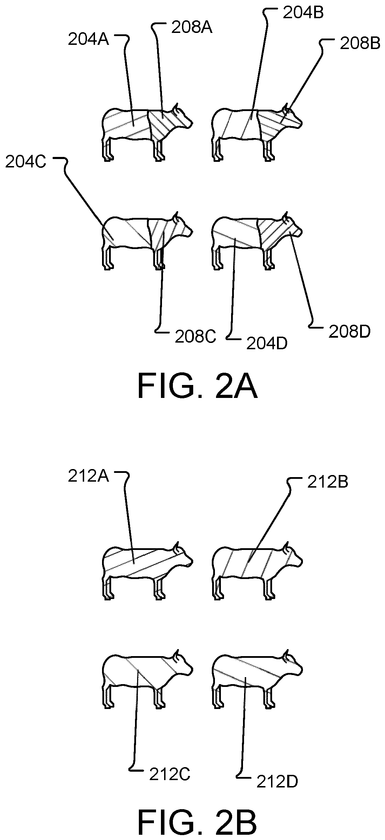

Figs. 2A-2B show the visual appearance of an illuminated object as seen by the human visual system (e.g., as appearing to the user) under both metamerically matched illumination and under incandescent illumination. The front of the cow and the back of the cow may have distinct spectral reflectance, such that general illumination of the cow with a broad spectrum of light will result in the front of the cow and the back of the cow reflecting a different color. For instance, as shown in Fig. 2A, general incandescent illumination (e.g., not metamerically matched illumination) may result in first colors 204A-204D being reflected from the back of the cow and second colors 208A-208D being reflected from the front of the cow. In contrast, Fig. 2B shows an illumination using metamerically matched illuminants. With metamerically matched illuminants, the human visual system cannot distinguish between the reflected colors of the front and the back of the cow, despite the front and back of the cow having distinct spectral reflectance. Indeed, the front and back of the cow appear as single colors 212A-212D to the user. In other words, using the metamerically matched, reflected light from the back and front of the cow can appear visually similar or the same to the user, but may be illuminated with spectrally different wavelengths of light.

In this illustration, the front and back of the cow include material properties which cause distinct spectral reflectances. A human view cannot distinguish the regions under metamerically matched illumination but can distinguish them under incandescent illumination (e.g., a broad spectrum, not metamerically matched illumination).

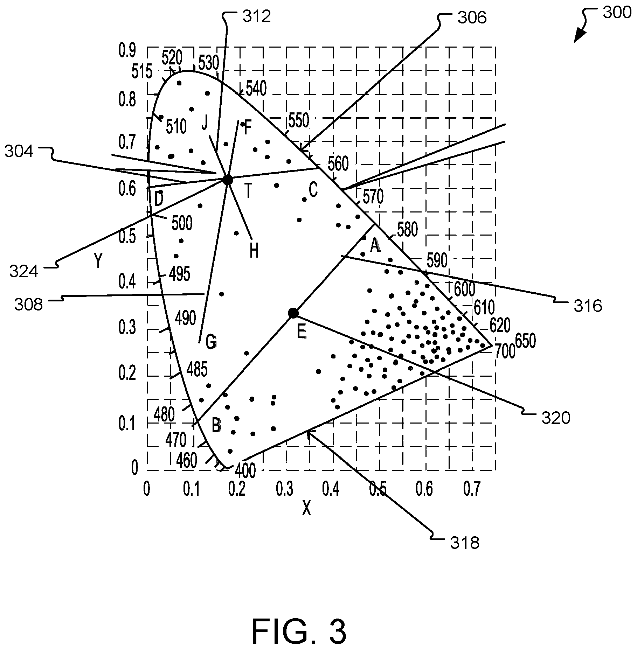

Fig. 3 shows a known chromaticity diagram 300 which shows a range of visible colors perceptible by a human eye. Any color in the diagram 300 could be selected and the system 100 could generate that selected color for the illuminating light using some combination of multiple illuminants within the other diagram. The system 100 may use various combinations of illuminants that when combined, metamerically match such that the human eye cannot perceive a difference in color. For example, the solid outline of the chromaticity diagram 300 encompasses all hues perceivable to the normal human eye (e.g., the human visual system). The horseshoe-shaped curve 306 contains the spectral colors, while the straight line at the bottom of the chromaticity diagram 300 is a line of purples 318.

The combination of light wavelengths to produce a given perceived color is not unique. For instance, the color 324 represented by the point T on the chromaticity diagram 300 may be produced based on a combination of pair CD 304, pair FG 308, and pair JH 312. In fact, any pair of colors points along the lines CD, FG, and JH may be combined, using different intensities, to produce the color 324 at the point T. In other words, the pair CD 304, the pair FG 308, and the pair JH 312 can each produce the color T if combined in the right proportions (e.g., as determined by the metamerism algorithm 106). While every point within the curve represents a unique perceivable hue, there are multiple combinations that may be used to produce that hue.

The chromaticity diagram 300 also comprises an achromatic point 320, shown as a point E on the chromaticity diagram 300. This point may be considered "white" for this given chromaticity diagram. The pair AB 316, or any other pair for which the connecting line passes through the point E, can form a complementary color pair. In some embodiments, the metamerism algorithm 106 may use the chromaticity diagram 300 (or any other chromaticity diagram) in determining the metamerically matched illuminants necessary to reduce flicker or strobing when sequencing through a plurality of illuminants.

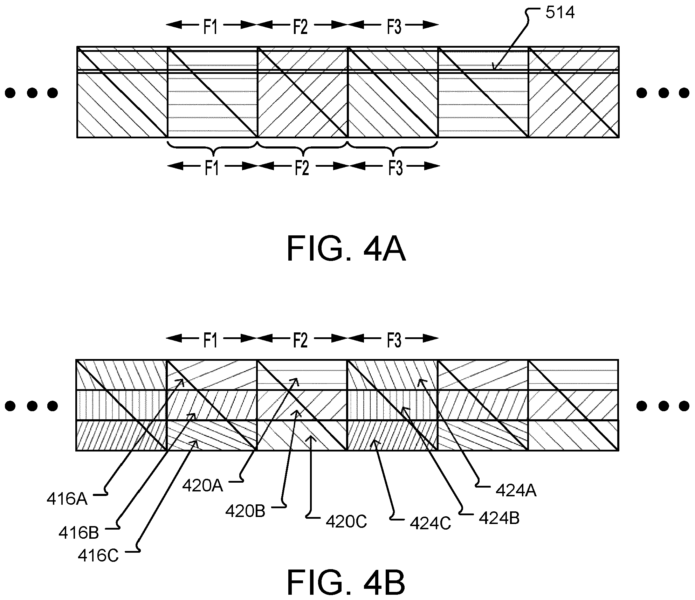

In some embodiments, the illumination shone on the anatomical element 116 may be a red-green-blue illuminant sequence. As shown in Fig. 4A, a red-green-blue illumination sequence may be superimposed over rolling shutter scan lines. Each sloped parallelogram corresponds to sensor lines 514 integrating for overlapping, but temporally offset time periods. Each sensor line may integrate for the entire frame interval. For example, a first frame F1 may correspond to a period of time that starts when red light is switched on and green and blue light are switched off, a second frame F2 may correspond to a period of time that starts when green light is switched on and red and blue lights are switched off, and a third frame F3 may correspond to a period of time that starts when blue light is switched on and red and green lights are switched off. Due to the rolling shutter (e.g., a rolling shutter common in a CMOS sensor which may be an element of the exoscope camera 108) the first read line of the first frame F 1 will contain entirely red light, but the last line of the first frame F1 will be almost entirely green as the integration period comes just a moment before the start of the second frame F2. Halfway through the first frame F1, the sensor lines 514 will contain fifty percent red light and fifty percent green light. A similar trend may occur in each of the second frame F2 and the third frame F3. That is, in the second frame F2 fades from entirely green light on the first line to blue light on the last line and the third frame F3 fades from blue light on the first line to red light on the last line.

By adding the first frame F 1, the second frame F2, and the third frame F3 using image processing produces an equivalent to the natural appearance of the illuminated scene under white light. In some embodiments, the frames may be interpolated to adjust to the correct time period (e.g., such that the frames displayed on the display 124 temporally match the actual illumination of the anatomical element 116).

In some embodiments, the addition to reconstruct the frame can be explained mathematically as:

$$[(1\text{-}w)^*R + w^*G] + [(1\text{-}w)^*G + w^*B] + [(1\text{-}w)^*B + w^*R] = R + G + B$$

where each of the three terms in square brackets correspond to the first frame F 1, the second frame F2, and the third frame F3, respectively. For instance, the first square bracket term may correspond to first frame F1, where the weight "w" begins at w=0 at the first line and ends at w=1 at the last line. As such, in the first frame F1 the "1-w" terms fade from 100% red light in the first line to 0% red light in the last line of the first frame F 1 while the "w" terms fade from 0% green light in the first line to 100% green light in the last line of the first frame F 1. The weight "w" may similarly begin at w=0 at the first line and ends at w=1 at the last line for the second frame F2 and the third frame F3. While the mathematical equation above demonstrates addition to reconstruct the frame, any linear combination of illuminates can be decorrelated into isolated spectra bands (such as the R, G, and B bands) by solving the appropriate system of equations. While the changing from the first frame F 1 to the second frame F2 and from the second frame F2 to the third frame F3 generates different illumination, the changing of the frames may still generate a strobing effect.

In some embodiments, there may be a gap in sensor integration due to differences in operation between the shutter scan and the read scan (e.g., there is a temporal spacing between the shutter moving and the reader reading the information captured by the shutter movement). This gap may result in a darker area. However, this effect may be compensated for by calculating how much light is present (or how much is missing during the blanking interval) and applying digital gain to compensate. An example of techniques that disclose how to compensate the blanking interval that can be used with any one or more of the embodiments disclosed herein is reference by Derek Bradley et al. Synchronization and Rolling Shutter Compensation for Consumer Video Camera Arrays, Computational Imaging Group, University of British Columbia, 2009.

To generate a spectrally distinct, metamerically matched, white illumination, three spectrally distinct white light illuminants can be created from a superposition of different sets of primaries (e.g., different sets of red, green, and blue light). The primaries may be chosen such that the illuminants in the sequence are perceptually indistinguishable from each other or greatly reduced in apparent contrast so as to reduce user perceived flicker or strobing. Unlike the previous example, where the R, G, and B strobing pattern would be visible to the surgeon (e.g., the surgeon would see the illuminants switch from red to green to blue to red and so forth), in this example the three white light illuminants appear similar. As with the previous example, the R, G, B image can be constructed through adding the images, but in this example each of the R, G, B components may be the sum of additional colors: $R = R1 + R2 + R3$; $G = G1 + G2 + G3$; and $B = B1 + B2 + B3$. In other words, each of the three color channels is formed as a superposition of related but distinct color primaries. While the sum of additional colors is shown here, any linear combination of the additional colors is also possible.

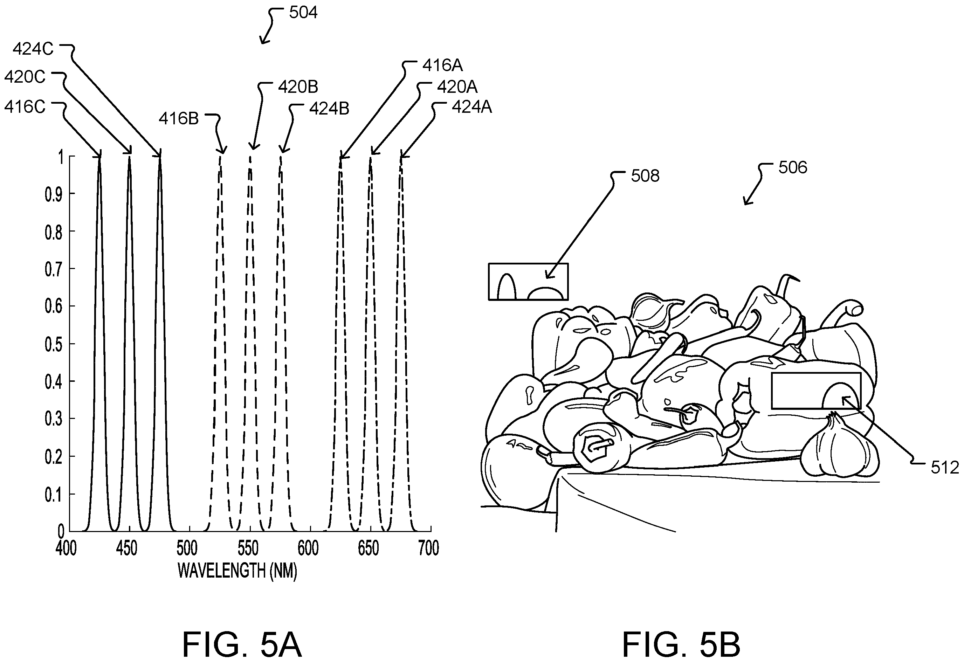

The construction of the additional color primaries may be performed by the metamerism algorithm 106. In some embodiments, the metamerism algorithm 106 may determine spectrally distinct primaries (e.g., using the chromaticity diagram 300), such as three separate subsets. As shown in Fig. 5A, a spectrum 504 may include a first red primary color 416A (corresponding to R1 in the sum above), a second red primary color 420A (corresponding to R2 in the sum above), and a third red primary color 424A (corresponding to R3 in the sum above); a first green primary color 416B (corresponding to G1 in the sum above), a second green primary color 420B (corresponding to G2 in the sum above), and a third green primary color 424B (corresponding to G3 in the sum above); and a first blue primary color 416C (corresponding to B1 in the sum above), a second blue primary color 420C (corresponding to B2 in the sum above), and a third blue primary color 424C (corresponding to B3 in the sum above). Such colors may be used in illuminating the anatomical element 116. For instance, three different subsets {R1, G1, B1}, {R2, G2, B2}, and {R3, G3, B3} may be weighted, mixed, blended, or otherwise appropriately balanced to form similar white illuminants (e.g., the metamerism algorithm 106 adjusts the amplitudes of R1, G1, and B1 such that the illuminants appear as white light). It is to be understood that, while the colors of Fig. 5A are shown as spectrally narrow, in general and in some embodiments of the present disclosure, there are many possible combinations of different illuminants (e.g., broadband sources) that may be used to cause the appearance of white light and that could be decorrelated for hyperspectral imaging through, for example, a linear transformation.

In some embodiments, the exoscope camera 108 or other component of the system 100 may comprise a color camera (e.g., a Bayer color filter array, a three-chip prism) which may be used to detect the different colors of the various illuminants found in the metamerically matched illumination.

Fig. 5B shows a scene 506 illuminated by white illuminant generated by amplitude adjustment of metamerically matched red, green, and blue light. While the scene appears to be illuminated by white light, the scene is illuminated by distinct spectral bands such that additional information can be extracted and presented to a user (e.g., via a display 124). For instance, a first portion 508 of the scene 506 may appear purple, and the underlying blue and red illuminants that are reflected by the first portion 508 may be shown on the display 124 (e.g., a plot of the wavelength and amplitudes of the blue and red illuminants). Similarly, a second portion 512 of the scene 506 may appear as red, and a plot of the underlying wavelength and amplitude of the red light reflected from the scene 506 may be rendered to the display 124. Stated differently, the sets of illuminants (e.g., underlying red, blue, and green illuminants) that make up the first portion 508 may be weighted (e.g., with the red and blue light having a greater weight than the green light), blended or mixed (e.g., with a greater amount of red and blue light used), or otherwise appropriately balanced to create the appearance of purple. Similarly, the set of illuminants that make up the second portion 512 may be weighted (e.g., with the red light having greater weight than the blue and green light), blended or mixed (e.g., with the red light in greater abundance than the blue and green light), or otherwise appropriately balanced to create the appearance of red.

It is possible for spectrally distinct reflective objects to appear similar under a first illuminant, but dissimilar under a second illuminant different from the first illuminant. Similarly, a spectrally uniform object may appear to change color if the illuminant is changed. As such, the perceived flicker of the surgical site may be optimized (e.g., the illuminants may be chosen such that the light reflected off one or more objects in the surgical site is metamerically matched) instead of optimizing for the perceived flicker of the illuminant (e.g., the flicker generated by the exoscope camera 108). This may be beneficial in exoscopic use cases, where it may be desired that certain tissues (e.g., red tissues) in an open surgical site maintain their appearance to a user (e.g., a surgeon), even as the illuminant is cycled.

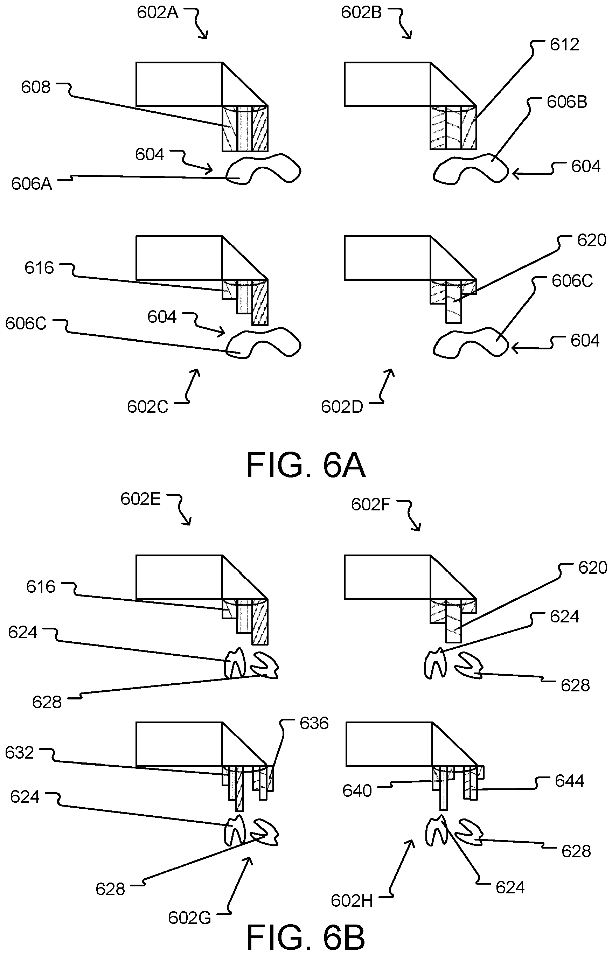

Figs. 6A-6B illustrate illuminations of anatomical elements according to at least one embodiment of the present disclosure.

Under a first illumination 602A and a second illumination 602B, a portion of an anatomical element 604 (which may be similar to or the same as the anatomical element 116) may be illuminated by two different illuminants. In other words, in the first illumination 602A, a first set of illuminants 608 may shine on a portion of the anatomical element 604, such that the portion of the anatomical element 604 appears as a first color 606A, while in the second illumination 602B, a second set of illuminants 612 may shine on a portion of the anatomical element 604, such that the portion of the anatomical element 604 appears a second color 606B. In some embodiments, the anatomical element 604 may appear as different colors under the different illuminations. However, the illuminants may be metamerically matched, such that the illuminants appear as the same or substantially similar colors to reduce the user-perceived flicker or strobing of the lights. Additionally or alternatively, the illuminants reflected off the portion of the anatomical element 604 may be metamerically matched, such that the user-perceived flicker or strobing of the light reflected off the anatomical element 604 (or a portion thereof) is minimized.

Under a third illumination 602C and a fourth illumination 602D, the anatomical element 604 may be illuminated by two different sets of illuminants that are metamerically matched such that the anatomical element 604 appears as a third color 606C under both sets of illuminants. Under the third illumination 602C, the anatomical element 604 may be illuminated by a third set of illuminants 616 such that the anatomical element 604 appears as the third color 606C. Similarly, under the fourth illumination 602D, the anatomical element 604 may be illuminated by a fourth set of illuminants 620. The fourth set of illuminants 620 may be metamerically matched such that the fourth set of illuminants 620 are spectrally different or distinct from the third set of illuminants 616 but result in the anatomical element 604 appearing as the third color 606C under both sets of illuminants. In some embodiments, the third set of illuminants 616 and/or the fourth set of illuminants 620 may be amplitude adjusted (e.g., using the metamerism algorithm 106) based on feedback from, for example, the exoscope camera 108 such that the appearance of the anatomical element 604 appears to be a continuous color (e.g., at the start of shining the illuminants on the anatomical site, the illuminants may not be metamerically matched but, based on feedback of the color appearance of the anatomical site captured by the exoscope camera, the illuminants are amplitude adjusted such that the color of the anatomical site temporally appears to be a continuous color).

In some embodiments, the surgical environment may include multiple anatomical elements (e.g., different tissues), making it difficult to stabilize the appearance of all anatomical elements (or portions thereof) using a single set of illuminants. For instance, under a fifth illumination 602E and a sixth illumination 602F, the illumination of a first object 624 (e.g., a surgical object or instrument, an anatomical element, etc.) may cause the first object (or a portion thereof) to reflect a continuous color, but a second object 628 or a portion thereof (e.g., a surgical object or instrument, an anatomical element, etc.) may not appear to reflect a continuous color. In other words, the light reflecting off the first object 624 may be metamerically matched when illuminated by the third set of illuminants 616 and the fourth set of illuminants 620, while the light reflecting off the second object 628 may not be metamerically matched, such that the second object 628 appears as a first color under the third set of illuminants 616 and a second color different from the first color under the fourth set of illuminants 620. To address this deficiency, the exoscope camera 108 may include multiple independently adjustable light-emitting elements (e.g., separate arrays of LEDs), with each light-emitting element capable of being correlated with and targeted to each of the first object 624 and the second object 628 (and/or any other element in the scene or surgical environment). Such independence of the light-emitting elements may enable the stabilization of multiple object colors simultaneously. For instance, in the seventh illumination 602G, a fifth set of illuminants 632 may be used to illuminate the first object 624, while a sixth set of illuminants 636 may be used to illuminate the second object 628. The sixth set of illuminants 636 may be a different set of primary colors (e.g., different hues of red, green, and/or blue light) than the fifth set of illuminants 632, such that the first object 624 appears as a first color and the second object 628 appears as a second color. Based on the feedback captured by the exoscope camera 108 and processed by the metamerism algorithm 106, the amplitudes may be adjusted such that in an eighth illumination 602H, a seventh set of illuminants 640 is used to illuminate the first object 624, while an eighth set of illuminants 644 is used to illuminate the second object 628. Due to the amplitude adjustment and the metameric matching of the illuminants, the first object 624 may remain the first color even though the seventh set of illuminants 640 is spectrally distinct from the fifth set of illuminants 632. Similarly, the second object 628 may remain the second color under the eighth set of illuminants 644, even though the illuminants have changed from the sixth set of illuminants 636. As such, at least one embodiment of the present disclosure enables multiple objects to be color stabilized using independently adjustable light-emitting elements.

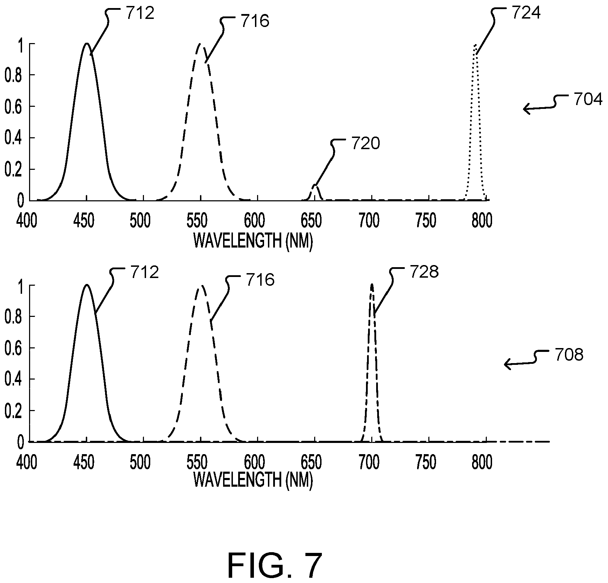

Fig. 7 illustrates a first set of spectra 704 and a second set of spectra 708. The first set of spectra 704 includes a blue light component 712 (e.g., light with a wavelength of 450 nm), a green light component 716 (e.g., light with a wavelength of 550 nm), a first red light component 720 (e.g., light with a wavelength of 650 nm), and a near-infrared component 724 (e.g., a non-visible light component with a wavelength of 790 nm). In some embodiments, the first red light component 720 may have a smaller amplitude relative to the blue light component 712 and/or the green light component 716.

In the second set of spectra 708, the blue light component 712 and the green light component 716 remain unchanged. However, now the near-infrared component 724 is turned off, and the first red light component 720 is replaced with a second red light component 728 (e.g., light with a wavelength of 700 nm). The second red light component 728 may be on the edge of the sensing abilities of the human visual system, such that the second red light component 728 does not appear as an intense or bright light. Such replacement of the first red light component 720 and the near-infrared component 724 by the second red light component 728 may permit the exoscope camera 108 to produce light such that the perception of red strobing is mitigated. Additionally, the alternation between the first red light component 720 and the near-infrared component 724 and the second red light component 728 may enable the system 100 to determine the contributions of the near-infrared component 724 on the overall fluorescence while also reducing the appearance of flickering or strobing of the illuminant source by metamerically matching the illuminants.

The system 100 may enable or carry out a method 800. The method 800 may be used, for example, to generate metamerically matched light for the purposes of illuminating an illumination site (e.g., an anatomical element, a surgical site, an object such as a surgical object or instrument, etc.).

The method 800 (and/or one or more steps thereof) may be carried out or otherwise performed, for example, by at least one processor. The at least one processor may be the same as or similar to the camera control unit(s) 104 described above. The at least one camera control unit may be part of the exoscope camera 108, or of any other component of the system 100. A camera control unit other than any camera control unit described herein may also be used to execute the method 800. The at least one camera control unit may perform the method 800 by executing elements stored in a memory. The elements stored in memory and executed by the processor may cause the processor to execute one or more steps of the steps of method 800. One or more portions of a method 800 may be performed by the camera control unit 104 executing the metamerism algorithm 106 and using one or more results thereof to carry out any one or more steps of the method 800.

The method 800 starts and then proceeds to step 804, which comprises receiving video data depicting a first illumination site. The video data may be raw data captured by and received from the exoscope camera 108. The video data may include information that, when executed or processed by the camera control unit 104, can be rendered to the display 124 to show the illuminated surgical site. The surgical site may be illuminated by the exoscope camera 108 using, for example, light generated by the light source 120, passed through a light guide, and shone onto the illumination site. The illumination site includes a first object (e.g., an anatomical element, a surgical object such as a surgical instrument or tool, etc.) to be illuminated by the light shone thereon. In some embodiments, the method 800 may begin the illumination of the first object using a predetermined set of color spectra (e.g., a first red light component, a first green light component, and a first blue light component).

The method 800 continues to step 808, which comprises determining, based on the video data, an illumination requirement. The determining may include calculating, using the metamerism algorithm 106, the required illumination. For instance, the metamerism algorithm 106 may determine that a first illumination is required. In some embodiments, the illumination requirements may change based on, for example, the type of surgical procedure, the type of imaging device (e.g., type of exoscope camera 108) used in the surgical procedure, combinations thereof, and/or the like.

The method 800 continues to step 812, where the metamerism algorithm 106 may select (e.g., from a database, using the chromaticity diagram 300, etc.) a first set of metameric illuminants. The first set of metameric illuminants may comprise a red light component, a green light component, and a blue light component. In some embodiments, the first set of metameric illuminants may include additional subsets of metamerically matched spectra (e.g., three subsets), such that each frame of the exoscope camera 108 shines a different spectral combination of the light on the surgical site (e.g., to cause different light reflectance of the surgical site). In such embodiments, each subset may also be metamerically matched.

After the metameric illuminants have been selected, the method 800 continues to step 816, where the metamerism algorithm 106 determines component amplitudes for each of the distinct spectra selected to form a metameric illumination. As an example, the metamerism algorithm 106 may determine that setting the red light component to a first amplitude, the green light component to a second amplitude, and the blue light component to a third amplitude may cause the spectra, when used as part of a sequence of metameric illuminants, to appear to a user (e.g., a surgeon) as a white light.

The method 800 continues to step 820, where the first set of metameric illuminants is shown on the first illumination site. For instance, the first set of metameric illuminants may be generated by the light source 120 and channeled through a light guide into the exoscope camera 108. The exoscope camera 108 may project the metameric illuminants on the first illumination site (e.g., an anatomical element, a surgical object, etc.). As discussed herein, the metameric illuminants may result in the first illumination sight appearing to the user as being illuminated by a first white light source.

The method 800 continues to step 824, where the first set of metameric illuminants are sequenced through (e.g., by the camera control unit 104) to a second set of metameric illuminants. For instance, the camera control unit 104 may change the components amplitudes and/or the wavelengths of the underlying illuminants, such that the underlying spectra illuminating the surgical site change. In some embodiments, the sequencing may be synchronized with the video timing of the image sensor. As such, the sequencing through the illuminants from the first set of metameric illuminants to the second set of metameric illuminants may result in a white light illuminating the surgical site, even as the underlying amplitude and/or wavelength components of the illuminants change from the first set to the second set.

The method continues to step 828, where the first illumination site is rendered to a display such as the display 124. The rendering of the illumination site may include capturing a live video feed (using, for example, the exoscope camera 108 or other imaging device) and sending the video feed to the camera control unit 104. The camera control unit 104 may then process the video feed and render the results to the display 124. The rendering of the display may enable a user (e.g., a physician or surgeon) to view the first illumination site being illuminated by the first set of metameric illuminants and, as the illuminants are sequenced through, the second set of metameric illuminants. The method 800 may then end or cycle through again to choose a third set of metameric illuminants such that the first illumination site appears to the user as being illuminated by the same or substantially similar white light source as the first white light source.

Any of the steps, functions, and operations discussed herein can be performed continuously and automatically.

To avoid unnecessarily obscuring the present disclosure, the preceding description omits a number of known structures and devices. This omission is not to be construed as a limitation of the scope of the claimed disclosure. Specific details are set forth to provide an understanding of the present disclosure. It should, however, be appreciated that the present disclosure may be practiced in a variety of ways beyond the specific detail set forth herein.

Furthermore, while the exemplary embodiments illustrated herein show the various components of the system collocated, certain components of the system can be located remotely, at distant portions of a distributed network, such as a LAN and/or the Internet, or within a dedicated system. Thus, it should be appreciated, that the components of the system can be combined into one or more devices, such as a server, communication device, or collocated on a particular node of a distributed network, such as an analog and/or digital telecommunications network, a packet-switched network, or a circuit-switched network. It will be appreciated from the preceding description, and for reasons of computational efficiency, that the components of the system can be arranged at any location within a distributed network of components without affecting the operation of the system.

Furthermore, it should be appreciated that the various links connecting the elements can be wired or wireless links, or any combination thereof, or any other known or later developed element(s) that is capable of supplying and/or communicating data to and from the connected elements. These wired or wireless links can also be secure links and may be capable of communicating encrypted information. Transmission media used as links, for example, can be any suitable carrier for electrical signals, including coaxial cables, copper wire, and fiber optics, and may take the form of acoustic or light waves, such as those generated during radio-wave and infra-red data communications.

While the flowcharts have been discussed and illustrated in relation to a particular sequence of events, it should be appreciated that changes, additions, and omissions to this sequence can occur without materially affecting the operation of the disclosed embodiments, configuration, and aspects.

A number of variations and modifications of the disclosure can be used. It would be possible to provide for some features of the disclosure without providing others.

Although the present disclosure describes components and functions implemented in the embodiments with reference to particular standards and protocols, the disclosure is not limited to such standards and protocols. Other similar standards and protocols not mentioned herein are in existence and are considered to be included in the present disclosure. Moreover, the standards and protocols mentioned herein and other similar standards and protocols not mentioned herein are periodically superseded by faster or more effective equivalents having essentially the same functions. Such replacement standards and protocols having the same functions are considered equivalents included in the present disclosure.

The present disclosure, in various embodiments, configurations, and aspects, includes components, methods, processes, systems and/or apparatus substantially as depicted and described herein, including various embodiments, subcombinations, and subsets thereof. Those of skill in the art will understand how to make and use the systems and methods disclosed herein after understanding the present disclosure. The present disclosure, in various embodiments, configurations, and aspects, includes providing devices and processes in the absence of items not depicted and/or described herein or in various embodiments, configurations, or aspects hereof, including in the absence of such items as may have been used in previous devices or processes, e.g., for improving performance, achieving ease, and/or reducing cost of implementation.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more embodiments, configurations, or aspects for the purpose of streamlining the disclosure. The features of the embodiments, configurations, or aspects of the disclosure may be combined in alternate embodiments, configurations, or aspects other than those discussed above. This method of disclosure is not to be interpreted as reflecting an intention that the claimed disclosure requires more features than are expressly recited in each claim. Rather, as the following claims reflect, inventive aspects lie in less than all features of a single foregoing disclosed embodiment, configuration, or aspect. Thus, the following claims are hereby incorporated into this Detailed Description, with each claim standing on its own as a separate preferred embodiment of the disclosure.

Moreover, though the description of the disclosure has included description of one or more embodiments, configurations, or aspects and certain variations and modifications, other variations, combinations, and modifications are within the scope of the disclosure, e.g., as may be within the skill and knowledge of those in the art, after understanding the present disclosure. It is intended to obtain rights, which include alternative embodiments, configurations, or aspects to the extent permitted, including alternate, interchangeable and/or equivalent structures, functions, ranges, or steps to those claimed, whether or not such alternate, interchangeable and/or equivalent structures, functions, ranges, or steps are disclosed herein, and without intending to publicly dedicate any patentable subject matter.

LIST OF REFERENCE NUMERALS

| | |
|---|---|
| 100 | System |
| 104 | camera control unit |
| 106 | metamerism algorithm |
| 108 | exoscope camera |
| 112 | illuminants |
| 112A | first illuminant |
| 112B | second illuminant |
| 112C | third illuminant |
| 116 | anatomical element |
| 120 | light source |
| 124 | display |
| 204A-D | First colors |
| 208A-D | second colors |
| 212A-D | single colors |
| 300 | chromaticity diagram showing range of colors perceptible by a human eye |
| 304 | pair CD |
| 306 | curve containing spectral colors |
| 308 | pair FG |
| 312 | pair JH |
| 316 | pair AB |
| 318 | line of purples |
| 320 | achromatic point |
| 324 | individual color |
| 416A | first red primary color |
| 416B | first green primary color |
| 416C | first blue primary color |
| 420A | second red primary color |
| 420B | second green primary color |
| 420C | second blue primary color |
| 424A | third red primary color |
| 424B | third green primary color |
| 424C | third blue primary color |
| 504 | spectrum |
| 506 | scene |
| 508 | first portion of scene 506 |
| 512 | second portion of scene 506 |
| 514 | sensor lines |
| 602A | first illumination |
| 602B | second illumination |
| 602C | third illumination |
| 602D | fourth illumination |
| 602E | fifth illumination |
| 602F | sixth illumination |
| 602G | seventh illumination |

| | |
|---|---|
| 602H | eighth illumination |
| 604 | anatomical element |
| 606A | first color |
| 606B | second color |
| 606C | third color |
| 608 | first set of illuminants |
| 612 | second set of illuminants |
| 616 | third set of illuminants |
| 620 | fourth set of illuminants |
| 624 | first object |
| 628 | second object |
| 632 | fifth set of illuminants |
| 636 | sixth set of illuminants |
| 640 | seventh set of illuminants |
| 644 | eighth set of illuminants |
| 704 | first set of spectra |
| 708 | second set of spectra |
| 712 | blue light component |
| 716 | green light component |
| 720 | first red light component |
| 724 | near-infrared component |
| 728 | second red light component |
| 800 | method |
| 804 | method step |
| 808 | method step |
| 812 | method step |
| 816 | method step |
| 820 | method step |
| 824 | method step |
| 828 | method step |
| F1 | first frame |
| F2 | second frame |
| F3 | third frame |

**Claims**

1. A system (100) to illuminate a first surgical site, the system (100) comprising:

   a light source (120);
   a processor and a controller (104) that determines a metamerically matched illumination, the metamerically matched illumination including a first set of illuminants (608) and a second set of illuminants (612) that, when temporally sequenced and at least partially superimposed, appears as a continuous white light; and
   an imaging device (108) in communication with the controller (104) that by means of the light source (120) emits the metamerically matched illumination on the first surgical site.

2. The system (100) of claim 1, wherein the first set of illuminants (608) comprises a first red light spectra (416A, 420A, 424A, 720, 728), a first green light spectra (416B, 420B, 424B, 716), and a first blue light spectra (416C, 420C, 424C, 712).

3. The system (100) of either claims 1 or 2, further comprising:
   a display device (124) configured to display the illuminated first surgical site.

4. The system (100) of any of the preceding claims, further comprising:
   a memory storing instructions thereon that, when executed by the processor, cause the processor to:
   determine a first amplitude of the first red light spectra (416A, 420A, 424A, 720, 728), a first amplitude of the first green light spectra (416B, 420B, 424B, 716), and a first amplitude of the first blue light spectra (416C, 420C, 424C, 712) to generate a first white light.

5. The system (100) of claim 4, wherein the light source (120) is designed to:

   receive the first amplitudes from the processor; and
   generate, based on the first amplitudes, the first white light.

6. The system (100) of any of the preceding claims, further comprising:
   a light guide, the light guide configured to guide the continuous white light from the light source (120) to the imaging device (108).

7. The system (100) of any of the preceding claims, wherein the first set of illuminants (608) and the second set of illuminants (612) appear as the continuous white light to a user.

8. The system (100) of any of the preceding claims, wherein the second set of illuminants (612) comprises a second red light spectra (416A, 420A, 424A, 720, 728), a second green light spectra (416B, 420B, 424B, 716), and a second blue light spectra (416C, 420C, 424C, 712).

9. The system (100) of claim 8, wherein the instructions further cause the processor to:
   determine a second amplitude of the second red light spectra (416A, 420A, 424A, 720, 728), a second amplitude of the second green light spectra (416B, 420B, 424B, 716), and a second amplitude of the second blue light spectra (416C, 420C, 424C, 712) to generate a second white light.

10. The system (100) of any of the preceding claims, wherein the light source (120) is further designed to:

    receive the second amplitudes from the processor; and
    generate, based on the second amplitudes, the second white light.

11. The system (100) of any of the preceding claims, wherein the instructions further cause the processor to:
    temporally sequence from the first set of illuminants to the second set of illuminants.

12. Use of the system (100) of any claim 1 - 11 to illuminate the first surgical site.

13. A system to illuminate a first surgical site (100), the system (100) comprising:

    a light source (120);
    a processor and a controller (104) that determines a metamerically matched reflectance, the metamerically matched reflectance including a first set of illuminants (608) and a second set of illuminants (612) that, when temporally sequenced, at least partially superimposed, and shone on the first surgical site, reflectively appear as a continuous white light; and
    an imaging device (108) in communication with the controller (104) that by means of the light source (120) emits the first set of illuminants (608) and the second set of illuminants (612) on the first surgical site.

14. The system (100) of claim 13, wherein the first set of illuminants (608) comprises a first red light spectra (416A, 420A, 424A, 720, 728), a first green light spectra (416B, 420B, 424B, 716), and a first blue light spectra (416C, 420C, 424C, 712), and wherein the second set of illuminants (612) comprises a second red light spectra (416A, 420A, 424A, 720, 728), a second green light spectra (416B, 420B, 424B, 716), and a second blue light spectra (416C, 420C, 424C, 712).

15. A method for illuminating a first surgical site comprising:

    determining (808) an illumination requirement, wherein an illumination requirement is a metamerically matched illumination or reflectance;
    determining (812), based on the illumination requirement, a first set of illuminants;
    determining, based on the illumination requirement, a second set of illuminants;
    generating the first set of illuminants or the second set of illuminants using a light source;
    causing (820) the first set of illuminants to be shone on a first illumination site;
    temporally sequencing (824) from the first set of illuminants to the second set of illuminants; and
    causing the second set of illuminants to be shone on the first illumination site, wherein the first set of illuminants and the second set of illuminants appear as a continuous white light.

**Patentansprüche**

1.  System (100) zum Beleuchten einer ersten Eingriffsstelle, wobei das System (100) Folgendes umfasst:

    eine Lichtquelle (120);
    einen Prozessor und eine Steuerung (104), die eine metamerisch angepasste Beleuchtung bestimmt, wobei die metamerisch angepasste Beleuchtung einen ersten Satz von Leuchtmitteln (608) und einen zweiten Satz von Leuchtmitteln (612) beinhaltet, die, wenn sie zeitlich aufeinander folgen und mindestens teilweise überlagert werden, als ein kontinuierliches Weißlicht erscheinen; und
    eine Bildgebungsvorrichtung (108), die mit der Steuerung (104) in Verbindung steht und mittels der Lichtquelle (120) die metamerisch angepasste Beleuchtung auf die erste Eingriffsstelle abgibt.

2.  System (100) nach Anspruch 1, wobei der erste Satz von Leuchtmitteln (608) ein erstes Rotlichtspektrum (416A, 420A, 424A, 720, 728), ein erstes Grünlichtspektrum (416B, 420B, 424B, 716) und ein erstes Blaulichtspektrum (416C, 420C, 424C, 712) umfasst.

3.  System (100) nach einem der Ansprüche 1 oder 2, ferner umfassend:
    eine Anzeigevorrichtung (124), die so konfiguriert ist, dass sie die beleuchtete erste Eingriffsstelle anzeigt.

4.  System (100) nach einem beliebigen der vorhergehenden Ansprüche, ferner umfassend:
    einen Speicher, auf dem Anweisungen gespeichert sind, die, wenn sie von dem Prozessor ausgeführt werden, den Prozessor veranlassen zum:
    Bestimmen einer ersten Amplitude des ersten Rotlichtspektrums (416A, 420A, 424A, 720, 728), einer ersten Amplitude des ersten Grünlichtspektrums (416B, 420B, 424B, 716) und einer ersten Amplitude des ersten Blaulichtspektrums (416C, 420C, 424C, 712), um ein erstes Weißlicht zu erzeugen.

5.  System (100) nach Anspruch 4, wobei
    die Lichtquelle (120) konzipiert ist zum:

    Empfangen der ersten Amplituden von dem Prozessor; und
    Erzeugen, anhand der ersten Amplituden, des ersten Weißlichts.

6.  System (100) nach einem beliebigen der vorhergehenden Ansprüche, ferner umfassend:
    einen Lichtleiter, wobei der Lichtleiter so konfiguriert ist, dass er das kontinuierliche Weißlicht von der Lichtquelle (120) zu der Bildgebungsvorrichtung (108) leitet.

7.  System (100) nach einem der vorhergehenden Ansprüche, wobei der erste Satz von Leuchtmitteln (608) und der zweite Satz von Leuchtmitteln (612) einem Benutzer als kontinuierliches Weißlicht erscheinen.

8.  System (100) nach einem der vorhergehenden Ansprüche, wobei der zweite Satz von Leuchtmitteln (612) ein zweites Rotlichtspektrum (416A, 420A, 424A, 720, 728), ein zweites Grünlichtspektrum (416B, 420B, 424B, 716) und ein zweites Blaulichtspektrum (416C, 420C, 424C, 712) umfasst.

9.  System (100) nach Anspruch 8, wobei die Anweisungen den Prozessor ferner veranlassen zum:
    Bestimmen einer zweiten Amplitude des zweiten Rotlichtspektrums (416A, 420A, 424A, 720, 728), einer zweiten Amplitude des zweiten Grünlichtspektrums (416B, 420B, 424B, 716) und einer zweiten Amplitude des zweiten Blaulichtspektrums (416C, 420C, 424C, 712), um ein zweites Weißlicht zu erzeugen.

10. System (100) nach einem der vorhergehenden Ansprüche, wobei die Lichtquelle (120) ferner konzipiert ist zum:

    Empfangen der zweiten Amplituden von dem Prozessor; und
    Erzeugen, anhand der zweiten Amplituden, des zweiten Weißlichts.

11. System (100) nach einem der vorhergehenden Ansprüche, wobei die Anweisungen den Prozessor ferner veranlassen zum:
    zeitlichen aufeinander Folgen von dem ersten Satz von Leuchtmitteln auf den zweiten Satz von Leuchtmitteln.

12. Verwendung des Systems (100) nach einem beliebigen der Ansprüche 1-11 zum Beleuchten der ersten Eingriffs-

stele.

13. System zum Beleuchten einer ersten Eingriffsstelle (100), wobei das System (100) Folgendes umfasst:

eine Lichtquelle (120);
einen Prozessor und eine Steuerung (104), die ein metamerisch angepasstes Reflexionsvermögen bestimmt, wobei das metamerisch angepasste Reflexionsvermögen einen ersten Satz von Leuchtmitteln (608) und einen zweiten Satz von Leuchtmitteln (612) beinhaltet, die, wenn sie zeitlich aufeinander folgen, mindestens teilweise überlagert werden und auf die erste Eingriffsstelle strahlen, reflektierend als ein kontinuierliches Weißlicht erscheinen; und
eine Bildgebungsvorrichtung (108), die mit der Steuerung (104) in Verbindung steht und mittels der Lichtquelle (120) den ersten Satz von Leuchtmitteln (608) und den zweiten Satz von Leuchtmitteln (612) auf die erste Eingriffsstelle abgibt.

14. System (100) nach Anspruch 13, wobei der erste Satz von Leuchtmitteln (608) ein erstes Rotlichtspektrum (416A, 420A, 424A, 720, 728), ein erstes Grünlichtspektrum (416B, 420B, 424B, 716) und ein erstes Blaulichtspektrum (416C, 420C, 424C, 712) umfasst, und wobei der zweite Satz von Leuchtmitteln (612) ein zweites Rotlichtspektrum (416A, 420A, 424A, 720, 728), ein zweites Grünlichtspektrum (416B, 420B, 424B, 716) und ein zweites Blaulichtspektrum (416C, 420C, 424C, 712) umfasst.

15. Verfahren zum Beleuchten einer ersten Eingriffsstelle, umfassend:

Bestimmen (808) einer Beleuchtungsanforderung, wobei eine Beleuchtungsanforderung eine metamerisch angepasste Beleuchtung oder ein Reflexionsgrad ist;
Bestimmen (812), anhand der Beleuchtungsanforderung, eines ersten Satzes von Leuchtmitteln;
Bestimmen, anhand der Beleuchtungsanforderung, eines zweiten Satzes von Leuchtmitteln;
Erzeugen des ersten Satzes von Leuchtmitteln oder des zweiten Satzes von Leuchtmitteln unter Verwendung einer Lichtquelle;
Bewirken (820), dass der erste Satz von Leuchtmitteln auf eine erste Beleuchtungsstelle gestrahlt wird;
zeitliches aufeinander Folgen von dem ersten Satz von Leuchtmitteln auf den zweiten Satz von Leuchtmitteln; und
Bewirken, dass der zweite Satz von Leuchtmitteln auf die erste Beleuchtungsstelle gestrahlt wird, wobei der erste Satz von Leuchtmitteln und der zweite Satz von Leuchtmitteln als ein kontinuierliches Weißlicht erscheinen.

## Revendications

1. Système (100) destiné à éclairer un premier champ opératoire, le système (100) comprenant :

une source (120) de lumière ;
un processeur et un dispositif de commande (104) permettant de déterminer un éclairage adapté de manière métamérique, l'éclairage adapté de manière métamérique comportant un premier ensemble de sources lumineuses (608) et un second ensemble de sources lumineuses (612), lesquels, lorsqu'ils sont séquencés temporellement et superposés au moins partiellement, sont perçus comme une lumière blanche continue ; et
un appareil d'imagerie (108) en communication avec le dispositif de commande (104), lequel, au moyen de la source (120) de lumière, émet l'éclairage adapté de manière métamérique sur le premier champ opératoire.

2. Le système (100) de la revendication 1, dans lequel le premier ensemble de sources lumineuses (608) comprend un premier spectre de lumière rouge (416A, 420A, 424A, 720, 728), un premier spectre de lumière verte (416B, 420B, 424B, 716), et un premier spectre de lumière bleue (416C, 420C, 424C, 712).

3. Le système (100) de l'une ou l'autre des revendications 1 ou 2, comprenant en outre :
un dispositif d'affichage (124) conçu pour afficher le premier champ opératoire éclairé.

4. Le système (100) de l'une quelconque des revendications précédentes, comprenant en outre :
une mémoire destinée à stocker des instructions, lesquelles, lors de leur exécution par le processeur, amènent le processeur :
à déterminer une première amplitude du premier spectre de lumière rouge (416A, 420A, 424A, 720, 728), une première amplitude du premier spectre de lumière verte (416B, 420B, 424B, 716), et une première amplitude du premier spectre de lumière bleue (416C, 420C, 424C, 712), afin de produire une première lumière blanche.

5. Le système (100) de la revendication 4, dans lequel
la source (120) de lumière est conçue :

pour recevoir les premières amplitudes à partir du processeur ; et
pour produire, sur la base des premières amplitudes, la première lumière blanche.

6. Le système (100) de l'une quelconque des revendications précédentes, comprenant en outre :
un guide de lumière, le guide de lumière étant conçu pour guider la lumière blanche continue de la source (120) de lumière à l'appareil d'imagerie (108).

7. Le système (100) de l'une quelconque des revendications précédentes, dans lequel le premier ensemble de sources lumineuses (608) et le second ensemble de sources lumineuses (612) sont perçus comme la lumière blanche continue par un utilisateur.

8. Le système (100) de l'une quelconque des revendications précédentes, dans lequel le second ensemble de sources lumineuses (612) comprend un second spectre de lumière rouge (416A, 420A, 424A, 720, 728), un second spectre de lumière verte (416B, 420B, 424B, 716), et un second spectre de lumière bleue (416C, 420C, 424C, 712).

9. Le système (100) de la revendication 8, dans lequel les instructions amènent en outre le processeur :
à déterminer une seconde amplitude du second spectre de lumière rouge (416A, 420A, 424A, 720, 728), une seconde amplitude du second spectre de lumière verte (416B, 420B, 424B, 716), et une seconde amplitude du second spectre de lumière bleue (416C, 420C, 424C, 712), afin de produire une seconde lumière blanche.

10. Le système (100) de l'une quelconque des revendications précédentes, dans lequel la source (120) de lumière est conçue en outre :

pour recevoir les secondes amplitudes à partir du processeur ; et
pour produire, sur la base des secondes amplitudes, la seconde lumière blanche.

11. Le système (100) de l'une quelconque des revendications précédentes, dans lequel les instructions amènent en outre le processeur :
à effectuer un séquençage temporel du premier ensemble de sources lumineuses au second ensemble de sources lumineuses.

12. Utilisation du système (100) de l'une quelconque des revendications 1-11 pour éclairer le premier champ opératoire.

13. Système (100) destiné à éclairer un premier champ opératoire, le système (100) comprenant :

une source (120) de lumière ;
un processeur et un dispositif de commande (104) permettant de déterminer une réflectance adaptée de manière métamérique, la réflectance adaptée de manière métamérique comportant un premier ensemble de sources lumineuses (608) et un second ensemble de sources lumineuses (612), lesquels, lorsqu'ils sont séquencés temporellement, superposés au moins partiellement et dirigés sur le premier champ opératoire, sont perçus de manière réfléchissante comme une lumière blanche continue ; et
un appareil d'imagerie (108) en communication avec le dispositif de commande (104), lequel, au moyen de la source (120) de lumière, émet le premier ensemble de sources lumineuses (608) et le second ensemble de sources lumineuses (612) sur le premier champ opératoire.

14. Le système (100) de la revendication 13, dans lequel le premier ensemble de sources lumineuses (608) comprend un premier spectre de lumière rouge (416A, 420A, 424A, 720, 728), un premier spectre de lumière verte (416B, 420B, 424B, 716), et un premier spectre de lumière bleue (416C, 420C, 424C, 712), et dans lequel le second ensemble de sources lumineuses (612) comprend un second spectre de lumière rouge (416A, 420A, 424A, 720, 728), un second spectre de lumière verte (416B, 420B, 424B, 716), et un second spectre de lumière bleue (416C, 420C, 424C, 712).

**15.** Procédé destiné à éclairer un premier champ opératoire, le procédé comprenant :

la détermination (808) d'un besoin d'éclairage, le besoin d'éclairage étant un éclairage ou une réflectance adaptés de manière métamérique ;

la détermination (812), sur la base du besoin d'éclairage, d'un premier ensemble de sources lumineuses ;

la détermination, sur la base du besoin d'éclairage, d'une second ensemble de sources lumineuses ;

la génération du premier ensemble de sources lumineuses ou du second ensemble de sources lumineuses à l'aide d'une source de lumière ;

la commande (820) du premier ensemble de sources lumineuses afin qu'il soit dirigé sur un premier site d'éclairage ;

le séquençage temporel (824) du premier ensemble de sources lumineuses au second ensemble de sources lumineuses ; et

la commande du second ensemble de sources lumineuses afin qu'il soit dirigé sur le premier site d'éclairage, le premier ensemble de sources lumineuses et le second ensemble de sources lumineuses étant perçus comme une lumière blanche continue.

FIG. 1

EP 4 205 692 B1

204A — 208A — 204B — 208B

204C —

208C — 204D — 208D

# FIG. 2A

212A — 212B

212C — 212D

# FIG. 2B

FIG. 3

EP 4 205 692 B1

FIG. 4A

FIG. 4B

FIG. 5A

FIG. 5B

EP 4 205 692 B1

FIG. 6A

FIG. 6B

FIG. 7

Start ⌐ 800

Receive video data depicting a first illumination site ⌐ 804

Determine, based on the video data, an illumination requirement ⌐ 808

Determine, based on the illumination requirement, a first set of metameric illuminations ⌐ 812

Determine a component amplitude for each illuminant in the first set of metameric illuminants ⌐ 816

Cause the first set of metameric illuminates to be shone on the first illumination site ⌐ 820

Sequence from the first set of metameric illuminants to a second set of metameric illuminants ⌐ 824

Cause the first illumination site to be rendered to a display ⌐ 828

End

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63294929
- US 20200305259 A1
- US 20080065345 A1
- US 20210075978 A1
- US 20050143662 A1

**Non-patent literature cited in the description**

- **DEREK BRADLEY et al.** *Synchronization and Rolling Shutter Compensation for Consumer Video Camera Arrays, Computational Imaging Group,* 2009